# EUROPEAN PATENT SPECIFICATION

(11) **EP 0 603 838 B1**
(45) Date of publication and mention of the grant of the patent: **12.03.1997**
(21) Application number: 93120636.1
(22) Date of filing: 21.12.1993
(51) Int. Cl.: D03D 15/02, A61N 1/16

(54) **Copper woven sheet for therapeutical use**
Gewebte Folie aus Kupfer für therapeutischen Gebrauch
Feuille tissée en cuivre pour usage thérapeutique

(30) Priority: 23.12.1992 IT MI922958
(43) Date of publication of application: 29.06.1994
(73) Proprietor: Gatti, Stefano, I-20146 Milano (IT)
(72) Inventor: Gatti, Stefano, I-20146 Milano (IT)
(74) Representative: Vatti, Paolo, Dr. Ing.

(56) References cited:
- DE-A- 3 442 369
- DE-U- 8 712 994
- FR-A- 2 455 870
- GB-A- 2 181 353

## Description

The present invention concerns a copper woven sheet for therapeutical use, i.e. a cloth interwoven with copper wire or copper plait.

As known, therapeutical properties and/or effects have been, and still are, attributed to copper when placed in contact with or near the human body.

Though no definite scientific explanations have so far been given in this respect, it has often been found that copper objects, when placed in direct contact with the human body or even close to it, have allowed to relieve pains, especially rheumatic ones or determined by inflammatory states, to eliminate or limit muscular contractions and spasms, and to give relief to patients affected by various syndromes, especially accompanied by pain.

This has led to the spreading of some types of objects allowing to keep copper close to and/or in contact with the human body, especially some of its parts, in a simple and comfortable way. Such objects mainly consist of bracelets essentially formed of a C-shaped copperplate strip to be applied on the wrists, of cardboard insoles lined with copper powder to be inserted between the feet and the shoes and, in the most interesting case, of the bed-clothing article incorporating a very thin, laminated, copper-containing flexible sheet material, described in GB-A-2181353.

Said objects - always having a thin sheet-type continuous surface of very limited weight and consistency - have proved to be somehow effective, but still show limited results and only on a fairly reduced percentage of patients.

It has now been found that - in a fully surprising way - far more significant results can be obtained with the copper woven article forming the therapeutical mean according to the present invention.

This therapeutical mean - provided for use as a myorelaxant, analgesic and anticephalalgic, intreating painful syndromes and inflammatory states of rheumatic origin - is characterized in that, it consists of a cloth interwoven with copper wire or copper plait weighing about 1500 g per sqm and having a size and consistency such as to ensure a steady and continuous contact with areas of the human body of not less than 1000 sq cm.

Though, as said, there is no thorough knowledge of the mechanisms enabling copper to produce its beneficial effects in reducing pain, it is deemed that they are somehow connected, on one hand, to the high electric and thermal conductivity of this metal and, on the other hand, to the electric and magnetic conditions of the patient and to an interaction between these two factors. It thus seems reasonable to think - even if no definite assumptions can be made, for the time being, on the nature and mechanisms by which copper acts - that a widespread, steady, continuous and long contact and/or closeness of copper to the patient's body should allow to obtain far more remarkable therapeutical effects than the discontinuous, irregular and brief contact, limited to some small body parts, provided by the systems normally adopted up to date.

Since the contact of the human body with a copper woven fabric - which can be made very soft and pliable, in the form of a heavy cloth - is no doubt quite continuous and intimate, and can be steadily kept over wide surfaces and for long periods of time, the surprising increase of the therapeutical properties of copper, guaranteed by the invention, finds a logical, even if partial and only empirical, explanation.

Preferably, the copper woven cloth forming the therapeutical mean according to the invention will be produced as a normal warp and weft fabric, interwoven with copper wire or copper plait (always using red electrolytic copper), hemmed on the four sides with a cotton band.

To fully profit by the characteristics of the invention, it has been found appropriate to produce copper woven cloths of the size of a bedsheet (160 x 180 cm, or 80 x 180 cm), of a towel (80 x 90 cm), or of a pillow cover (40 x 65 cm), so as to treat the whole body, the trunk and the head and/or the shoulders respectively. Nevertheless, the special copper woven sheet according to the invention can have considerable effects and provide important advantages even if cut into reduced sizes and shapes, for treating smaller areas of the human body: for instance, a typical example is to cut out from said sheet insoles to be interposed between the feet and the shoes; the consistency and structure of the sheet make in fact such insoles far more efficient than those - cited further above - used up to date, though the size of the sheet is in this case very limited.

The invention of course concerns also the therapeutical use of the aforespecified copper woven sheet and of the other objects made therewith or derived therefrom.

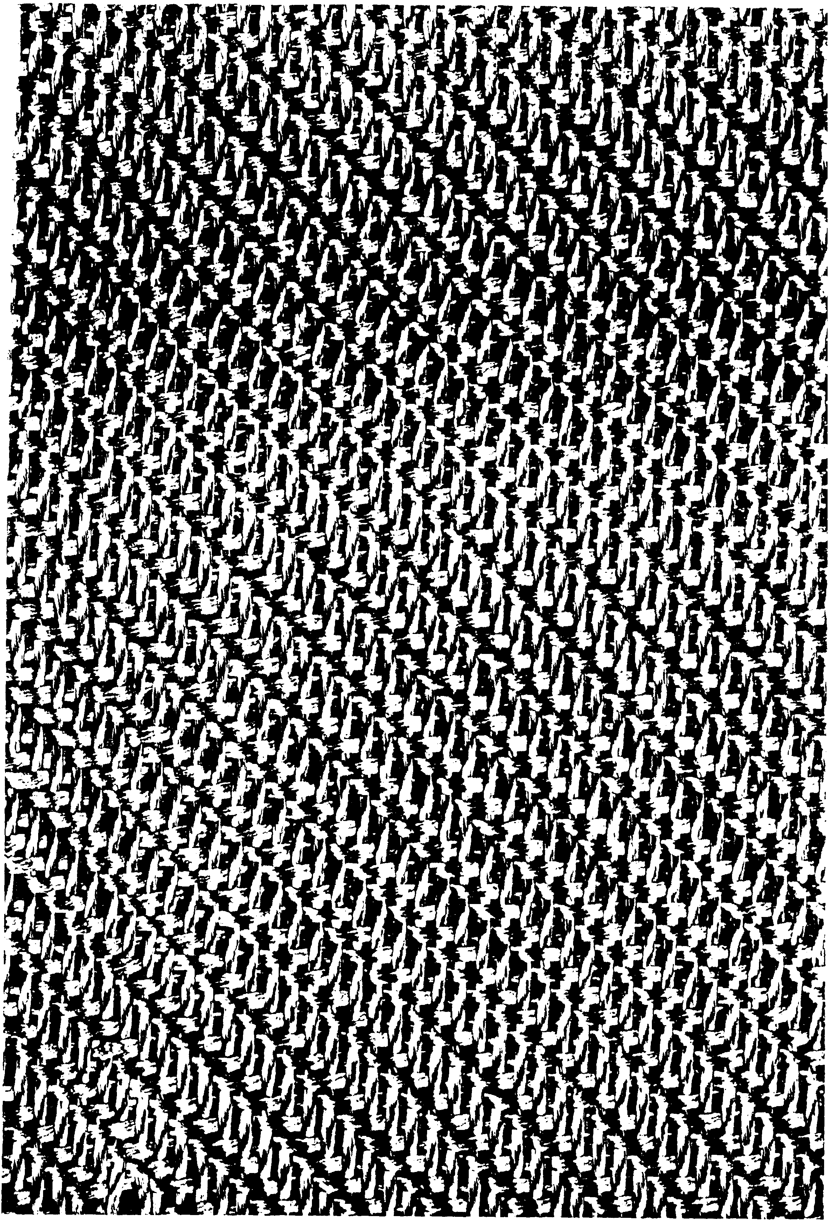

The invention will now be described in further detail, by mere way of example, with reference to a preferred embodiment thereof, illustrated on the accompanying drawing, which shows diagrammatically, on an enlarged scale, the structure of the fabric used to make the sheet according to the invention.

In the embodiment shown on the accompanying drawing, said sheet is obtained starting from a normal warp and weft woven cloth, produced with a copper plait having a diameter of about 0.7 mm and consisting of 36 wires, each with a diameter of about 0.1 mm. The weight of this cloth is of about 3 kg per sq m. The cloth is hemmed on the four sides with a cotton band, after having been cut into the wanted sizes.

As already said, the four sizes preferred for the sheet according to the invention are, more or less, 160 x 180 cm, 80 x 180 cm, 80 x 90 cm, 40 x 65 cm, and they are suited to treat the whole body, the trunk and the head and/or the shoulders respectively. Nevertheless, also other sizes can obviously be taken into consideration, according to the therapeutical requirements having to be satisfied.

The copper cloth according to the invention can also be cut into different shapes - other than the normal rectangular shape of a sheet - to form insoles for shoes, inner linings for hats, knee-pads, shoulder covers, and bands of any type.

To accomplish their therapeutical function, the sheets and other objects of copper woven cloth described heretofore should be used in direct contact with, or in close proximity to the body of the patient, or to parts thereof, for prolonged periods of time. Thus, the bed-size sheets can be positioned, on the single or double bed, under the usual cotton or linen sheet, to accomplish their function while the patient is asleep, or anyhow lying on the bed. The same can be done with the towlor pillow-size sheets, which can however be used also when sitting on a chair, or standing, for instance while getting ready. The other objects can be even worn by the patient, for example together with the usual clothing articles, with which they can be combined.

Experience has already proved that many patients have felt much better when using the sheet according to the invention: it mainly allows to reduce - sometimes even considerably - the painful symptoms, but it also allows to reduce, quite frequently, cramps and spasms (thereby relieving pain even further). Substantially, the sheet has a considerable myorelaxing and analgesic effect, and it hence provides an interesting support to the traditional pharmacological therapy. It is particularly effective in the case of pains and inflammatory states of rheumatic origin and in the case of headaches, especially due to muscular tension. In the summer season, it also has a refreshing effect which, added to the others, improves the comfort and relief of the patient.

It should also be noted that the structure of the sheet according to the invention, interwoven with copper wire or plait in the manner of a heavy cloth, ensures a very efficient perspiration, thereby contributing, also from this point of view, to the patient's comfort.

The sheet has finally proved to be unexpectedly helpful in treating spastic subjects, who have shown surprising improvements after its use. It is deemed that the myorelaxing action of the sheet is apt to significantly favour the progress of these patients during the normal treatments of various types to which they are subjected.

It is understood that other practical embodiments of the sheet according to the invention can be provided, differing from the one described, and that said sheet can be cut into the most different sizes and shapes, to be adapted to the most varied therapeutical uses.

## Claims

1. A therapeutical mean for use - as a myorelaxant, analgesic and anticephalalgic - in treating painful syndromes and inflammatory states of rheumatic origin, characterized in that, it consists of a cloth interwoven with copper wire or copper plait weighing about 1500 g per sqm and having a size and consistency such as to ensure a steady and continuous contact with areas of the human body of not less than 1000 sq cm.

2. A therapeutical mean as in claim 1) in which said copper woven cloth is produced as a normal warp and weft fabric, interwoven with copper wire or copper plait.

3. A therapeutical mean as in claim 1) and 2) in which said copper woven cloth is interwoven with a copper plait having a diameter of about 0.7 mm and constisting of 36 wires, each with a diameter of about 0.1 mm, and hemmed on the four sides with a cotton band.

4. A therapeutical mean as in claim 1) to 3) in which said copper woven cloth is interwoven with red electrolytic copper wire or copper plait.

5. A therapeutical mean as in claims 1) to 4), in which said copper woven cloth has the dimensions of a bedsheet (for instance 160 x 180 cm, or 80 x 180 com).

6. A therapeutical mean as in claims 1) to 4), in which said copper woven cloth has the dimensions of a towel (for instance 80 x 90 cm).

7. A therapeutical mean as in claims 1) to 4), in which said copper woven cloth has the dimensions of a pillow cover (for instance 40 x 65 cm).

## Patentansprüche

1. Ein therapeutisches Mittel zur Verwendung - zur Entspannung der Muskeln, zur Analgesie und zur Bekämpfung von Kopfschmerzen - bei der Behandlung schmerzhafter Syndrome und entzündlicher Zustände rheumatischen Ursprungs, dadurch gekennzeichnet, daß es aus einem Tuch besteht, in das ein Kupferdraht oder ein Kupfergeflecht eingewebt ist, mit einem Gewicht von 1.500 g pro qm² und mit einer Größe und Konsistenz, die einen ständigen und einen kontinuierlichen Kontakt mit den Flächen des menschlichen Körpers von nicht weniger als 1.000 cm² sicherstellt.

2. Ein therapeutisches Mittel nach Anspruch 1, wobei das Tuch, in das das Kupfer eingewebt ist, aus einem normalen Kett- und Schußgewebe besteht, in das ein Kupferdraht oder ein Kupfergeflecht eingewebt ist.

3. Ein therapeutisches Mittel nach einem der Ansprüche 1 oder 2, bei dem das Tuch, in das Kupfer eingewebt ist, mit einem Kupfergeflecht gewebt ist, das einen Durchmesser von etwa 0,7 mm hat und aus 36 Drähten besteht, jeder mit einem Durchmesser von 0,1 mm, und auf den vier Seiten von einem Baumwollband umgeben wird.

4. Ein therapeutisches Mittel nach einem der Ansprüche 1 bis 3, bei dem das Tuch, in das Kupfer eingewebt ist, mit einem roten elektrolytischen Kupferdraht oder - Kupfergeflecht verwebt ist.

5. Ein therapeutisches Mittel nach einem der Ansprüche 1 bis 4, bei dem das mit Kupfer gewebte Tuch die Erstreckungen eines Bettlakens hat (beispielsweise 160 x 180 cm oder 80 x 180 cm) hat.

6. Ein therapeutisches Mittel nach einem der Ansprüche 1 bis 4, bei dem das Tuch, in das Kupfer eingewebt ist, die Erstreckungen eines Handbuchs hat (beispielsweise 80 x 90 cm).

7. Ein therapeutisches Mittel nach einem der Ansprüche 1 bis 4, bei dem das Tuch, in das Kupfer eingewebt ist, die Erstreckungen eines Kopfkissenbezugs hat (beispielsweise 40 x 65 cm).

## Revendications

1. Article thérapeutique pour utilisation - comme myorelaxant, analgésique et anticéphalalgique - pour traiter les symptômes douloureux et les états inflammatoires d'origine rhumatismale, caractérisé en ce qu'il consiste en un textile entrelacé de fil de cuivre ou de tressage de cuivre pesant environ 1500 g par m² et ayant une taille et une consistance permettant d'assurer un contact régulier et continu avec des surfaces du corps humain d'au moins 1000 cm².

2. Article thérapeutique selon la revendication 1 dans lequel ledit textile tissé de cuivre est produit sous la forme d'un tissu normal à chaîne et à trame, entrelacé avec un fil de cuivre ou un tressage de cuivre.

3. Article thérapeutique selon les revendications 1 et 2 dans lequel ledit textile normal est entrelacé avec un tressage de cuivre ayant un diamètre d'environ 0,7 mm et constitué de 36 fils, ayant chacun un diamètre d'environ 0,1 mm, et ourlé sur les quatre côtés avec une bande de coton.

4. Article thérapeutique selon les revendications 1 à 3 dans lequel ledit textile tissé de cuivre est entrelacé de fil de cuivre électrolytique ou de tressage de cuivre.

5. Article thérapeutique selon les revendications 1 à 4, dans lequel ledit textile tissé de cuivre a les dimensions d'un drap de lit (par exemple 160 x 180 cm, ou 80 x 180 cm).

6. Article thérapeutique selon les revendications 1 à 4, dans lequel ledit textile tissé de cuivre a les dimensions d'une serviette de toilette (par exemple 80 x 90 cm).

7. Article thérapeutique selon les revendications 1 à 4 dans lequel ledit textile tissé de cuivre a les dimensions d'une taie d'oreiller (par exemple 40 x 65 cm).
